(19) Europäisches Patentamt
European Patent Office
Office européen des brevets

(11) Veröffentlichungsnummer: **0 296 488 B1**

(12) # EUROPÄISCHE PATENTSCHRIFT

(45) Veröffentlichungstag der Patentschrift: **27.11.91**

(51) Int. Cl.5: **C07C 45/59**, C07C 49/203, C07C 49/04

(21) Anmeldenummer: **88109632.5**

(22) Anmeldetag: **16.06.88**

(54) **Verfahren zur Herstellung von ungesättigten und gesättigten Ketonen.**

(30) Priorität: **24.06.87 DE 3720850**

(43) Veröffentlichungstag der Anmeldung:
**28.12.88 Patentblatt 88/52**

(45) Bekanntmachung des Hinweises auf die Patenterteilung:
**27.11.91 Patentblatt 91/48**

(84) Benannte Vertragsstaaten:
**BE DE FR GB NL**

(56) Entgegenhaltungen:
**DE-B- 1 109 669**
**DE-B- 1 142 350**

**THE JOURNAL OF ORGANIC CHEMISTRY, Band 49, Nr. 4, 1984, Seiten 701 - 703, American Chemical Society, Washington, US; G. CARDILLO et al.: "Novel Synthesis of alpha-Hydroxy Ketones and gamma-or delta-Keto Esters from Cyclic Iodo Carbonates and Iodo Lactones"**

**JOURNAL OF THE AMERICAN CHEMICAL SOCIETY, Band 103, Nr. 18, September 1981, Seiten 5414 -5417, American Chemical Society, Washington, US; T.B. CAMERON et al.: "Flash Vacuum Thermolysis of 1,3-Dioxolan-4-ones"**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Hupfer, Leopold, Dr.**
**Waltershöhe 3**
**W-6701 Friedelsheim(DE)**
Erfinder: **Schneider, Kurt, Dr.**
**Auf dem Koeppel**
**W-6702 Bad Duerkheim(DE)**

EP 0 296 488 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von ungesättigten und gesättigten Ketonen durch Decarboxylierung von in 4-Stellung substituierten 5-Methylen-1,3-dioxolanonen.

Ketone sind wegen ihrer vielseitigen Verwendungsmöglichkeit gesuchte chemische Verbindungen. Man verwendet gesättigte Ketone z.B. als Lösungsmittel in der Gummi- und Kunststoffindustrie, als Lösungsmittel bei chemischen Reaktionen, als Extraktionsmittel oder als Ausgangsstoffe für organische Reaktionen, z.B. als Zwischenprodukte für Farbstoffe, Pflanzenschutzmittel und Pharmaprodukte und als Duftstoffe.

Bifunktionelle Verbindungen sind wertvolle Bausteine für organische Synthesen. Zu dieser Verbindungsklasse gehören auch die $\alpha,\beta$-ungesättigten Ketone. Diese Ketone werden u.a. zur Synthese von Verbindung mit physiologischer Aktivität verwendet. Ein weiteres Anwendungsgebiet für die $\alpha,\beta$-ungesättigten Ketone, insbesondere für Methylisopropylenketon liegt in der Chemie von temperaturstabilen Polymeren und Copolymeren. Methylisopropenylketon wird als Monomeres für Poly(isopropenylmethylketon) oder als Copolymerisat mit Polybutadien bzw. PVC verwendet. Weiterhin finden derartige Ketone in der Herstellung von Epoxidharzen Verwendung.

Es ist bekannt, a,$\beta$-ungesättigte Ketone durch Aldolkondensation von aliphatischen Ketonen und Formaldehyd in Gegenwart von Oxalsäure bzw. in Gegenwart von Kationenaustauschern und $H_2O$ unter erhöhtem Druck (GB-PS 993 389 oder FR-PS 1 383 548) oder durch Reaktion von Enolacetaten mit Ketonen in Gegenwart von Lewis-Säuren wie $BF_3$, $TiCl_4$ oder durch Oxidation verzweigter Olefine in 50 %iger Essigsäure und in Gegenwart von $PdCl_2$ als Katalysator (BE-PS 658 801) oder durch Kondensation von gesättigten Carbonylverbindungen in Gegenwart von $BF_3$ herzustellen.

Daneben gibt es ein mehrstufiges Verfahren, bei dem man $\alpha,\beta$-ungesättigte Ketone durch Kondensation von Ketonen in Gegenwart von Tosylmethylisocyanid, anschließende Alkylierung und Hydrolyse erhält. Andere aufwendige Herstellverfahren sind z.B. die Ozonolyse von 2,3-Dimethylbutadienen oder die anodische Oxidation von $\beta$-Ketocarboxylaten oder die Photoisomerisation von 1,2-Diacylcyclobutanen. Die Gasphasenoxidation von Olefinen mit Metalloxid/Phosphoroxid-Katalysatoren führt zu einem Gemisch verschiedener Verbindungen, unter denen sich auch $\alpha,\beta$-ungesättigte Ketone befinden.

Zur Herstellung der gesättigten Ketone kann man die ungesättigten Ketone nach bekannten Methoden hydrieren.

Die bekannten Verfahren zur Herstellung von ungesättigten und damit auch von gesättigten Ketonen weisen verschiedene Nachteile auf, sei es, daß man von schwer zugänglichen Ausgangsverbindungen ausgeht, daß man toxische und korrosive Homogenkatalysatoren verwendet, oder daß man eine energieaufwendige oder mehrstufige Reaktionsführung wie Ozonolyse bzw. Photoisomerisation in Kauf nehmen muß.

Insbesondere bei der technischen Herstellung von asymmetrisch substituierten Ketonen ist man in der Regel auf die Kondensation unterschiedlicher organischer Säuren unter Decarboxilierung angewiesen, wie sie in der DE-OS 27 58 113 und in Houben-Weyl, 1973, Bd. 7/2a, Ketone I, S. 627 ff beschrieben ist. Bei diesem Verfahren ist der Zwangsanfall von symmetrisch substituierten Ketonen und Kohlendioxid nachteilig.

Ein großer Nachteil der Herstellung von gesättigten Ketonen aus Vorläufern der ungesättigten Ketone ist die zweistufige Verfahrensweise.

Es ist auch aus DE-PS 1 109 669 bekannt, daß man Dioxolanone zu ungesättigtgen Ketonen decarboxylieren kann. Dieses Verfahren wird bevorzugt thermisch in der Flüssigphase durchgeführt. Um einen glatten Reaktionsverlauf zu gewährleisten, muß man in Gegenwart von hochsiedenden Lösungsmitteln arbeiten. Diese müssen nach der Reaktion in einer aufwendigen Destillation wieder abgetrennt werden.

Daraus ergab sich die Aufgabe, ungesättigte und gesättigte Ketone, insbesondere asymmetrisch substituierte Ketone durch eine einfache Reaktion aus gut zugänglichen Ausgangsverbindungen bei kurzen Verweilzeiten mit hoher Selektivität und Raumzeitausbeute zu synthetisieren.

Es wurde nun gefunden, daß man die oben beschriebenen Nachteile vermeidet und ungesättigte Ketone der Formel (I)

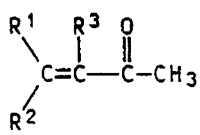

(I)

und gesättigte Ketone der Formel (II)

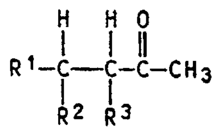

(II),

in der $R^1$, $R^2$, $R^3$ Wasserstoff, Alkylreste mit 1 bis 12 Kohlenstoffatomen, Cycloalkyl-, Aryl-, Aralkyl- bzw. Alkylarylreste, die ihrerseits substituiert sein können, bedeuten, bzw. $R^1$ und $R^3$ oder $R^2$ und $R^3$ zusammen mit den C-Atomen, an die sie gebunden sind, ein Cycloalken bzw Cycloalken bilden können, in einfacher Weise erhält, wenn man in 4-Stellung substituierte 5-Methylen-1,3-dioxolanone der Formel (III)

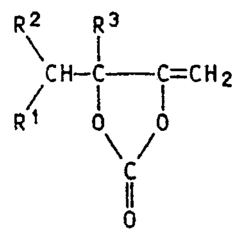

(III),

in der $R^1$ , $R^2$ und $R^3$ Wasserstoff, Alkyreste mit 1 bis 12 Kohlenstoffatomen, Cycloalkyl-, Aryl-, Aralkyl- bzw. Alkylarylreste, die ihrerseits substituiert sein können, bedeuten, bzw. $R^1$ und $R^3$ oder $R^2$ und $R^3$ zusammen mit den C-Atomen, an die sie gebunden sind, ein Cycloalkan, a) zur Herstellung von, ungesättigten Ketonen (I) in Gegenwart von Zeolithen, von Phoshaten mit Zeolithstruktur und/oder B-, Ce-, Fe-, Zr-, Sr-Phosphaten als Katalysatoren umsetzt und b) zur Herstellung von gesättigten Ketonen (II) in Gegenwart von Wasserstoff an Zeolithen, an Phosphaten mit Zeolithstruktur und/oder B-, Ce-, Fe, Zr-, Sr-Phosphaten, die zumindest eine Hydrierkomponente tragen, als Katalysatoren umsetzt. Geeignete Ausgangsstoffe sind z.B. folgende 5-Methylen-4,4-dialkyl-1,3-dioxolanone: 5-Methylen-4,4-dimethyl-1,3-dioxolanon-(2), 5-Methylen-4,4-diethyl-1,3-dioxolanon-(2), 5-Methylen-4-methyl-4-ethyl-1,3-dioxolanon-(2), 5-Methylen-4-methyl-4-(4'-methyl-penten-3'-dioxolanon-(2), 5-Methylen-4-methyl-4-butyl-1,3-dioxolanon-(2) und 5-Methylen-4-methyl-4-n-octyl-1,3-dioxolanon-(2). Ausgangsverbindungen, in denen $R^1$ und $R^3$ oder $R^2$ und $R^3$ zusammen für Alkylreste stehen, z.B. für den Propyliden-, Tetramethylen- oder Hexamethylenrest, sind beispielsweise 5-Methylen-4,4-spirohexamethylen-1,3-dioxolanon-(2) und 5-Methylen-4,4-spiropentamethylen-1,3-dioxolanon-(2).

Die substituierten Methylendioxolanone sind z.B. durch Umsetzung von Acetylenalkoholen mit Kohlendioxid in Gegenwart von Katalysatoren, wie z.B. Kupfersalzen, erhältlich.

Als Katalysatoren im Sinne der Erfindung sind im allgemeinen Zeolithe des Pentasiltyps wie Aluminiumsilikatzeolithe, Borosilikatzeolithe, Eisensilikatzeolithe und Zeolithe des Faujasittyps, die gegebenenfalls noch eine Hydrierkomponente tragen, geeignet.

Die Zeolithe können z.B. mit Alkalimetallen, Übergangsmetallen und mit Seltenen Erdmetallen, gegebenenfalls zusätzlich zur Hydrierkomponente, dotiert werden.

Man kann als Katalysatoren aber auch Phosphate der Elemente B, Ce, Zr, Fe, Sr oder deren Gemische verwenden. Auch hydrothermal hergestellte Phosphate sind z.B. als Katalysatoren geeignet, z.B. hydrothermal hergestellte Aluminiumphosphate, Siliciumaluminiumphosphate, Eisenaluminiumphosphate, Boraluminiumphosphate. Zur Herstellung der gesättigten Ketone tragen diese Katalysatoren noch mindestens eine Hydrierkomponente.

Als Katalysatoren für das erfindungsgemäße Verfahren verwendet man die Zeolithe zweckmäßig in der aciden Form.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kuboktaeders, der aus Vierringen bzw. Sechsringen aufgebaut ist. Je nach Verknüpfung der Kuboktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind auch Zeolithe aus der Mordenit-Gruppe oder engporige Zeolithe vom Erionit- bzw. Chabasit-Typ oder Zeolithe vom Faujasit-Typ, z.B. Y-, X-oder L-Zeolithe.

In diese Gruppe von Zeolithen gehören auch die sogenannten "ultrastabilen" Zeolithe des Faujasittyps, d.h. dealuminierte Zeolithe.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, , Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40 000. Aluminosilikatzeolithe des Pentasiltyps können auch in etherischem Medium wie Diethylenglykoldimethylether, in alkoholischem Medium wie Methanol bzw. 1,4-Butandiol oder in Wasser synthetisiert werden.

Zu den erfindungsgemäß verwendbaren siliciumreichen Zeolithen ($SiO_2/Al_2O_3 \geq 10$) gehören auch die verschiedenen ZSM-Typen, Ferrierit, Nu-1 und Silicalit®.

Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Die so hergestellten Alumino-, Boro- und Eisensilikatzeolithe können nach ihrer Isolierung, Trocknung bei 100 bis 160 °C und Calcinierung bei 450 bis 550 °C, vorzugsweise 500 °C , mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C /16 h getrocknet und bei 500 °C /16 h calciniert.

Man erhält auch vorteilhafte Katalysatoren, wenn z. B. der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Zweckmäßigerweise führt man diese Dotierung so durch, daß man den verformten Zeolithen in einem Steigrohr vorlegt und bei 20 bis 100 °C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung

schließt sich zumindest eine Trocknung, wahlweise eine abermalige Calcinierung an.

Eine mögliche Ausführungsform besteht darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Pd(NO_3)_2$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wird der getränkte Zeolith bei etwa 150° C getrocknet und bei etwa 550° C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100° C unter Rühren etwa 24 h aufzuschlämmen. Nach Abfiltrieren, Trocknen bei etwa 150° C und Calcinierung bei etwa 500° C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber eine wäßrige $Co(NO_3)_2$-, $Ni(NO_3)_2$-, $Fe(NO_3)_2$-Lösung oder ammoniakalische $Pd(NO_3)_2$-Lösung bei leicht erhöhter Temperatur zwischen 30 und 80° C im Kreislauf 15 bis 20 h leitet. Danach wird mit Wasser ausgewaschen, bei etwa 150° C getrocknet und bei etwa 550° C calciniert. Bei manchen metalldotierten Zeolithen, z.B. Pt-, Pd-, Cu-, Co-, Ni-dotierten Zeolithen ist eine Nachbehandlung mit Wasserstoff vorteilhaft.

Es ist auch in vielen Fällen vorteilhaft, den Katalysator vor Reaktionsbeginn zu reduzieren. Beispielsweise ein Pt, Pd, Co, Ni, Fe, Rh, Ru oder Co dotierten Zeolith-Katalysator wird im Reaktor auf 170-220° C unter $N_2$ aufgeheizt, dann wird langsam $H_2$ beigefügt. Die Temperatur wird so lange konstant gehalten bis kein $H_2O$ mehr kommt. Eine Reduziervorschrift ist auch in J. of Catal. 89, 520-526 (1984) beschrieben.

Durch Imprägnierung und Ionenaustausch können auch gleichzeitig mehrere Metalle als Hydrierkomponenten aufgebracht werden.

Um eine möglichst hohe Selektivität, hohe Umsätze sowie lange Standzeiten zu erreichen, kann es vorteilhaft sein, die Zeolithe zu modifizieren. Eine geeignete Modifizierung der Katalysatoren besteht z.B. darin, daß man zusätzlich zu den oben genannten Hydrierkomponenten den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Ba, Metalle der 3., 4. und 5. Hauptgruppe wie B, Al, Ga, Ge, Sn, Pb, Bi, Seltene Erdmetalle wie La, Ce, Pr, Nd, Er, Yb und U eingesetzt. Die Modifizierung mit diesen Metallen kann gleichzeitig mit der Aufbringung der Hydrierkomponente durch Ionenaustausch oder Imprägnierung erfolgen oder vor der Aufbringung der Hydrierkomponenten erfolgen oder sich an die Aufbringung der Hydrierkomponente und eventuell an eine nachfolgende Calcination anschließen.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. An diese Art der Modifizierung schließt sich dann die Aufbringung der Hydrierkomponenten an. Im einzelnen geht man vorteilhaft so vor, daß man Zeolithe in Pulverform mit 1 n Phosphorsäure 1 Stunde bei 80° C behandelt. Nach der Behandlung wird mit Wasser gewaschen, bei 110° C/16 Stunden getrocknet und bei 500° C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80° C mit einer 3 bis 25 Gew.%igen, insbesondere 12 bis 20 Gew.%igen wäßrigen Salzsäure. Anschließend wird der so behandelte Zeolith mit Wasser gewaschen, getrocknet und bei 400° C bis 500° C calciniert. Nunmehr erfolgt die Aufbringung der Hydrierkomponente durch Ionenaustausch bzw. Imprägnierung wie zuvor beschrieben.

Eine besondere Ausführungsform für die Säurebehandlung besteht darin, daß man das zeolithische Material vor seiner Verformung bei erhöhter Temperatur mit 0,001 n bis 2 n, vorzugsweise 0,05 n bis 0,5 n Flußsäure, behandelt, beispielsweise durch Erhitzen unter Rückfluß über einen Zeitraum von 0,5 bis 5, vorzugsweise 1 bis 3 Stunden. Nach Isolierung, z.B. durch Abfiltrieren und Auswaschen des zeolithischen Materials, wird dieses zweckmäßig bei Temperaturen von 100 bis 160° C getrocknet und bei Temperaturen von 450° C bis 600° C calciniert. Gemäß einer weiteren bevorzugten Ausführungsform für die Säurebehandlung, wird das zeolithische Material nach seiner Verformung mit Bindemittel zweckmäßig bei Temperaturen von 50 bis 90° C, insbesondere bei 60 bis 80° C, über einen Zeitraum von 0,5 bis 5 h, vorzugsweise mit 12 bis 20 Gew.%iger Salzsäure, behandelt. Zweckmäßig wird das zeolithische Material anschließend ausgewaschen, bei Temperaturen von 100 bis 160° C getrocknet und bei Temperaturen von 450 bis 600° C calciniert. Einer HF-Behandlung kann sich auch eine HCl-Behandlung anschließen. Nunmehr erfolgt die Aufbringung der Hydrierkomponente wie voranstehend angeführt.

Nach einer anderen Arbeitsweise kann man Zeolithe durch Aufbringen von Phosphorverbindungen, wie Trimethylphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert. Hieran schließt sich die Aufbringung der Hydrierkomponente an.

Wenn bei der erfindungsgemäßen Verwendung der zeolithischen Katalysatoren wie auch der anderen hierfür eingesetzten Katalysatoren eine durch Koksabscheidung bedingte Desaktivierung eintritt, empfiehlt es sich, diese durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Weitere Katalysatoren für die Herstellung von gesättigten und ungesättigten Ketonen sind Phosphate mit Zeolithstruktur, wie Aluminiumphosphate, Siliciumaluminiumphosphate, Siliciumeisenaluminiumphosphate, Eisenaluminiumphosphate, Boraluminiumphosphate oder deren Gemische.

Als Aluminiumphosphat-Katalysatoren werden für das erfindungsgemäße Verfahren, insbesondere unter hydrothermalen Bedingungen, synthetisierte Aluminiumphosphate eingesetzt. Geeignete Aluminiumphosphate sind z.B. APO-5, APO-9, APO-11, APO-12, APO-14, APO-21, APO-25, APO-31 und APO-33.

Beispielsweise $AlPO_4$-5 (APO-5) wird synthetisiert, indem man Orthophosphorsäure mit Pseudoboehmit (Catapal SB R) in Wasser homogen mischt zu dieser Mischung Tetrapropylammoniumhydroxid gibt und danach bei etwa 150 °C 20 bis 60 h unter autogenem Druck in einem Autoklaven umsetzt. Das abfiltrierte $AlPO_4$ wird bei 100 bis 160 °C getrocknet und bei 450 bis 550 °C calciniert.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei etwa 200 °C unter autogenem Druck während 200 bis 400 h synthetisiert. Verwendet man anstelle DABCO-Lösung Ethylendiamin, so gelangt man zu APO-12.

Die Synthese des $AlPO_4$-21 (APO-21) erfolgt aus Orthophosphorsäure und Pseudoboehmit in wäßriger Pyrrolidin-Lösung bei 150 bis 200 °C unter autogenem Druck während 50 bis 200 h.

Für das erfindungsgemäße Verfahren kann man auch bekannte Siliciumaluminiumphosphate wie SAPO-5, SAPO-11, SAPO-31, und SAPO-34 einsetzen. Diese Verbindungen werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C, während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C .

Als Siliciumaluminiumphosphate sind auch ZYT-5, ZYT-6, ZYT-7, ZYT-9, ZYT-11 und ZYT-12 geeignet.

Als weitere Phosphat-Katalysatoren kann man bei dem Verfahren gefällte Phosphate der Elemente B, Zr, Fe, Ce, Sr einsetzen.

Borphosphate als Katalysatoren für das erfindungsgemäße Verfahren kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650 °C vorzugsweise 300 bis 500 °C herstellen.

Cerphosphate als Katalysatoren kann man beispielsweise durch Fällung aus einer wäßrigen Cersalz-Lösung mit einem Alkaliphosphat erhalten.

Auf diese Phosphate, sowohl Phosphate mit Zeolithstruktur oder auch gefällte Phosphate, werden durch Imprägnierung (Tränken und Aufsprühen) oder in manchen Fällen auch durch Ionenaustausch die Hydrierkomponenten, wie voran beschrieben, aufgebracht. Auch kann wie bei den Zeolithkatalysatoren eine Modifizierung mit Metallen oder Säuren erfolgen. Auch hier schließt sich die Aufbringung der Hydrierkomponente an. Eine eventuelle Modifizierung mit Metallen oder Säuren ist vorher oder nachher möglich.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umwandlung in der Regel gewählten Reaktionsbedingungen sind in der bevorzugten Gasphase 100 bis 500 °C, z.B. 150 bis 450 °C und insbesondere 300 bis 400 °C und eine Katalysatorbelastung WHSV von 0,1 bis 20 $h^{-1}$g Ausgangsstoff je g Katalysator und Stunde. Die Reaktion wird vorzugsweise im Festbett oder im Wirbelbett durchgeführt.

Es ist auch möglich, die Reaktion in der Flüssigphase, in Suspensions-, Riesel- oder Sumpffahrweise,

bei Temperaturen zwischen 50 und 200 °C durchzuführen.

Das Verhältnis Wasserstoff zu Dioxolanon kann 1 bis 100 molar, insbesondere 3 bis 30 molar für die Herstellung der gesättigten Ketone sein.

Das Verfahren wird im allgemeinen bei Normaldruck oder je nach Flüchtigkeit der Ausgangsverbindung bei vermindertem oder erhöhtem Druck diskontinuierlich, vorzugsweise kontinuierlich durchgeführt.

Schwerflüchtige oder feste Ausgangsstoffe kann man in gelöster Form, z.B. in THF-, Toluol- oder Petrolether-Lösung einsetzen. Es ist auch eine Verdünnung des Ausgangsstoffes mit derartigen Lösungsmitteln oder mit Inertgasen wie $N_2$, Ar, $H_2O$-Dampf möglich.

Nach der Umsetzung werden die entstandenen Produkte direkt weiterverarbeitet oder durch übliche Verfahren, z. B. durch Destillation aus dem Reaktionsgemisch isoliert. Nichtumgesetzte Ausgangsstoffe werden gegebenenfalls in die Umsetzung zurückgeführt.

Beispiele 1 bis 20

Die Reaktion wird in der Gasphase unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch.

Für die Beispiele werden folgende Katalysatoren eingesetzt.

Katalysator A

Ein Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50 : 50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$. Daraus werden durch Verformen mit einem Verformungshilfsmittel 2 mm-Stränge hergestellt, die bei 110 °C/16 h getrocknet und bei 500 °C/24 h calciniert werden.

Katalysator B

Ein Aluminosilikatzeolith vom Pentasil-Typ wird unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50 : 50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Der Aluminosilikatzeolith enthält 96,1 Gew.% $SiO_2$ und 4,6 Gew.% $Al_2O_3$. Der Katalysator wird zu 2-mm-Strängen verformt, bei 110 °C/16 h getrocknet und bei 500 °C/24 h calciniert.

Katalysator C

Ein Eisensilikatzeolith des Pentasil-Typs wird unter hydrothermalen Bedingungen bei autogenem Druck und 165 °C aus 273 g Wasserglas, gelöst in 253 g einer wäßrigen 1,6-Hexandiamin-Lösung (Mischung 50 : 50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolithen mit einem $SiO_2/Fe_2O_3$-Verhältnis von 17,7 und einem $Na_2O$-Gehalt von 1,2 Gew.%. Der Zeolith wird mit hochdispersem $SiO_2$ im Gewichtsverhältnis 80 : 20 zu 2,5-mm-Strängen verstrangt, bei 110 °C/16 h getrocknet und bei 500 °C/24 h calciniert. Danach wird mit einer 20 %igen $NH_4Cl$-Lösung bei 80 °C ionenausgetauscht, solange bis der Na-Gehalt nach der Trocknung bei 110 °C und Calcination bei 500 °C/5 h 0,002 Gew.% beträgt.

Katalysator D

Katalysator D erhält man, indem man Katalysator A mit $Ce(NO_3)_3$-Lösung imprägniert, danach bei 130 °C/2 h trocknet und bei 540 °C/2 h calciniert. Der Ce-Gehalt beträgt 1,7 Gew.%.

Katalysator E

$AlPO_4$-5 (APO-5) wird synthetisiert, indem man 200 g 98 %ige Phosphorsäure und 136 g Boehmit in 335 g Wasser löst bzw. suspendiert, hierzu 678 g einer 30 %igen wäßrigen Tetrapropylammoniumhydroxid-Lösung zugibt und diese Mischung in einem Rührautoklaven bei 150 °C während 43 Stunden unter autogenem Druck umsetzt. Nach Abfiltrieren wird das kristalline Material bei 120 °C getrocknet und bei 500 °C/16 h calciniert. Das so synthetisierte $AlPO_4$-5 enthält 46,5 Gew.% $P_2O_5$ und 45,5 Gew.% $Al_2O_3$. Dieses Material wird mit Boehmit im Gewichtsverhältnis 60 : 40 zu 2-mm-Strängen verformt, abermals bei 120 °C getrocknet und bei 500 °C/8 h calciniert.

Katalysator E

$AlPO_4$-12 (APO-12) wird synthetisiert, indem man 200 g 98 %ige Phosphorsäure und 136 g Boehmit in 721 g Wasser löst bzw. suspendiert, hierzu 30 g Ethylendiamin zugibt und diese Mischung in einem Rührautoklaven bei 200 °C während 24 Stunden unter autogenem Druck umsetzt. Nach Abfiltrieren wird das kristalline Material bei 120 °C getrocknet und bei 500 °C/16 h calciniert. Das so synthetisierte $AlPO_4$-12 enthält 56,1 Gew.% $P_2O_5$ und 43,1 Gew.% $Al_2O_3$. Dieses Material wird mit Boehmit im Gewichtsverhältnis 60 : 40 zu 2-mm-Strängen verformt, abermals bei 120 °C getrocknet und bei 500 °C/8 h calciniert.

Katalysator G

SAPO-5 wird aus einer Mischung aus 200 g 98 %ige Phosphorsäure, 136 g Boehmit, 60 g Kieselsol (30 %ig), 287 g Tripropylamin und 587 g $H_2O$ hergestellt. Diese Mischung wird bei 150 °C während 168 Stunden unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$. SAPO-5 wird mit einem Verformungshilfsmittel zu 3-mm-Strängen verformt, bei 120 °C getrocknet und bei 500 °C calciniert.

Katalysator H

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator H enthält 47,1 Gew.% Ce und 12,7 Gew.% P.

Katalysator I

Im Handel erhältliches Zirkonphosphat wird mit Verformungshilfsmitteln zu 2-mm-Strängen verformt, bei 110 °C getrocknet und bei 500 °C/16 h calciniert.

Katalysator J

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator J enthält 8,77 Gew.% B und 28,3 Gew.% P.

In der nachfolgenden Tabelle 1 sind die mit den voranbeschriebenen Katalysatoren A bis J erzielten Ergebnisse sowie die Reaktionsbedingungen aufgeführt. Die Proben wurden nach 6 Stunden Reaktionszeit analysiert. Nach längerem Stehen bildet sich das Dimere des Isopropenylmethylketons. Dies kann durch Zugabe z.B. von Hydrochinon und durch Kühlung unterdrückt werden.

Katalysator K

Katalysator K erhält man, indem man die Stränge von Katalysator A mit einer wäßrigen $Cu(NO_3)_2$-Lösung imprägniert, danach bei 130 °C/2 h trocknet und bei 540 °C/2 h calciniert. Der Cu-Gehalt beträgt 3,4 Gew.%.

Katalysator L

Die Stränge des Borosilikatzeolithen, wie bei Katalysator A beschrieben, werden in einer Kolonne vorgelegt und einem Ionenaustausch mit einer ammoniakalischen Palladiumnitrat-Lösung bei 50 °C unter-

worfen. Nach Auswaschen mit $H_2O$ wird bei 110 °C getrocknet und bei 500 °C/5 h calciniert. Der Pd-Gehalt beträgt 1,9 Gew.%.

Katalysator M

Katalysator M wird wie Katalysator K hergestellt, jedoch mit einer wäßrigen Lösung aus Pd- und Pr-Nitrat anstatt Cu-Nitrat getränkt. Der Pd-Gehalt beträgt 1,0 Gew.%, der Pr-Gehalt 4,6 Gew.%.

Katalysator N

Katalysator N wird erhalten, indem man Katalysator G, wie bei Katalysator L beschrieben, einem Ionenaustausch mit Palladiumnitrat unterwirft. Der Pd-Gehalt beträgt 1 Gew.%.

Die mit den Katalysatoren K - N in Gegenwart von Wasserstoff erhaltenen Versuchsergebnisse sind in Tabelle 2 zusammengestellt.

Katalysator O (Vergleichskatalysator)

Katalysator O ist ein gefälltes Aluminiumphosphat, das durch Fällung aus $Al(NO_3)_3$-$H_3PO_4$-Lösung mit $NH_3$ bei pH = 6 - 7 erhalten wird. Nach Abfiltrieren des Niederschlags wird bei 100 °C getrocknet und bei 500 °C calciniert. Katalysator O enthält 28,5 Gew.% Al und 13,2 Gew.% P.

Tabelle 1:

5-Methylen-4,4-dimethyl-1,3-dioxolan-2-on (I) $\xrightarrow{-CO_2}$ Methylisopropenylketon (II)

| Beispiel | 1 | 2 | 3 | 4 | 5 | 6 | 7 | 8 | 9 | 10 | 11 | 12 | 13 | 14 | 15[1)] | 16[1)] |
|---|---|---|---|---|---|---|---|---|---|---|---|---|---|---|---|---|
| Katalysator | A | A | A | E | E | B | C | E | F | G | H | I | J | J | O | O |
| Temperatur [°C] | 350 | 375 | 400 | 300 | 350 | 350 | 350 | 350 | 350 | 350 | 350 | 350 | 350 | 400 | 350 | 400 |
| WHSV $h^{-1}$ | 4 | 3 | 3 | 2 | 2 | 4 | 3 | 3 | 4 | 4 | 3 | 3 | 3 | 4 | 3 | 4 |
| Umsatz von I [%] | 67,9 | 84,8 | 97,4 | 80,7 | 98,0 | 80,2 | 94,4 | 85,0 | 74,3 | 76,4 | 99,6 | 98,5 | 71,7 | 98,0 | 94,0 | 98,6 |
| Selektivität [%] | | | | | | | | | | | | | | | | |
| II | 98,9 | 97,8 | 93,4 | 95,2 | 90,6 | 91,1 | 89,7 | 93,5 | 94,2 | 92,5 | 92,7 | 92,3 | 90,3 | 89,0 | 82,5 | 86,0 |
| III | - | - | - | - | 0,2 | 1,5 | 2,2 | 1,0 | 0,9 | 2,1 | | - | | | 1,1 | |

I 5-Methyl-4,4-dimethyl-1,3-dioxolan-2-on gelöst in THF (75 %ige Lösung)

II Methylisopropenylketon

III Dimeres von II

1) Vergleichsbeispiel

Tabelle 2:

5-Methylen-4,4-dimethyl-1,3-dioxolan-2-on (I) $\xrightarrow[-CO_2]{+H_2}$ Methylisopropenyl-keton (III)

| Beispiel | 17 | 18 | 19 | 20 |
|---|---|---|---|---|
| Katalysator | K | L | M | N |
| Temperatur [°C] | 400 | 350 | 400 | 350 |
| WHSV $h^{-1}$ | 4 | 4 | 4 | 4 |
| I : $H_2$ molar | 1:6 | 1:6 | 1:6 | 1:6 |
| Umsatz von I [%] | 100 | 90,1 | 100 | 73,0 |
| Selektivität [%] | | | | |
| III | 95,7 | 95,9 | 95,7 | 93,5 |

I 5-Methyl-4,4-dimethyl-1,3-dioxolan-2-on gelöst in THF (75 %ige Lösung)

## Patentansprüche

**1.** Verfahren zur Herstellung von ungesättigten Ketonen der Formel (I)

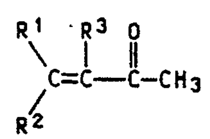

(I)

und gesättigten Ketonen der Formel (II)

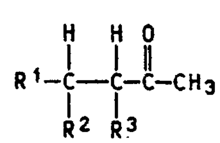

(II),

in der $R^1$, $R^2$, $R^3$ Wasserstoff, Alkylreste mit 1 bis 12 Kohlenstoffatomen, Cycloalkyl-, Aryl-, Aralkyl- bzw. Alkylarylreste, die ihrerseits substituiert sein können, bedeuten, bzw. $R^1$ und $R^3$ oder $R^2$ und $R^3$ zusammen mit den C-Atomen, an die sie gebunden sind, ein Cycloalkan bzw Cycloalken bilden können, dadurch gekennzeichnet, daß man in 4-Stellung substituierte 5-Methylen-1,3-dioxolanone der Formel (III)

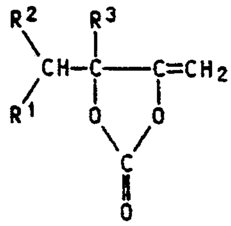

(III),

in der $R^1$, $R^2$ und $R^3$ Wasserstoff, Alkylreste mit 1 bis 12 Kohlenstoffatomen, Cycloalkyl-, Aryl-, Aralkyl- bzw. Alkylarylreste, die ihrerseits substituiert sein können, bedeuten, bzw. $R^1$ und $R^3$ oder $R^2$ und $R^3$ zusammen mit den C-Atomen, an die sie gebunden sind, ein Cycloalkan, a) zur Herstellung von ungesättigten Ketonen (i)in Gegenwart von Zeolithen, von Phosphaten mit Zeolithstruktur und/oder B-, Ce-, Fe-, Zr-, Sr-Phosphaten als Katalysatoren umsetzt und b) zur Herstellung von gesättigten Ketonen (II) in Gegenwart von Wasserstoff an Zeolithen, an Phosphaten mit Zeolithstruktur und/oder B-, Ce-, Fe-, Zr-, Sr-Phosphaten, die zumindest eine Hydrierkomponente tragen, als Katalysatoren umsetzt.

2. Verfahren nach Anspruch 1, dadurch gekennzeichnet, daß man als Katalysatoren Zeolithe des Pentasiltyps, die gegebenenfalls noch eine Hydrierkomponente tragen, verwendet.

3. Verfahren nach Anspruch 1, dadurch gekennzeichnet, daß man als Katalysatoren Aluminiumsilikatzeolithe des Faujasittyps, die gegebenenfalls noch eine Hydrierkomponente tragen, verwendet.

4. Verfahren nach den Ansprüchen 1 bis 3, dadurch gekennzeichnet, daß man Katalysatoren verwendet, die mit Alkalimetallen, Erdalkalimetallen, Übergangsmetallen oder mit Seltenen Erden, gegebenenfalls zusätzlich zur Hydrierkomponente, dotiert sind.

5. Verfahren nach Anspruch 1, dadurch gekennzeichnet, daß man als Phosphate mit Zeolithstruktur hydrothermal hergestellte Aluminiumphosphate oder Siliciumaluminiumphosphate oder Siliciumeisenaluminiumphosphate oder Eisenaluminiumphosphate oder Boraluminiumphosphate, die gegebenenfalls noch eine Hydrierkomponente tragen, verwendet.

6. Verfahren nach den Ansprüchen 1 bis 6, dadurch gekennzeichnet, daß man die Umsetzung in der Gasphase ausführt.

## Claims

1. A process for the preparation of an unsaturated ketone of the formula (I)

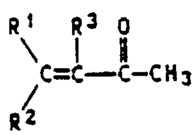

(I)

or a saturated ketone of the formula (II)

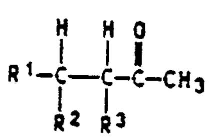

(II)

where $R^1$, $R^2$ and $R^3$ are each hydrogen, alkyl of 1 to 12 carbon atoms or a cycloalkyl, aryl, aralkyl or alkylaryl radical which in turn may be substituted, or $R^1$ and $R^3$ or $R^2$ and $R^3$, together with the carbon atoms to which they are bonded, may form a cycloalkane or cycloalkene, wherein a 5-methylene-1,3-dioxolanone which is substituted in the 4-position and is of the formula (III)

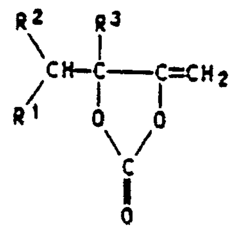

where $R^1$, $R^2$ and $R^3$ are each hydrogen, alkyl of 1 to 12 carbon atoms or a cycloalkyl, aryl, aralkyl or alkylaryl radical which in turn may be substituted or $R^1$ and $R^3$ or $R^2$ and $R^3$, together with the carbon atoms to which they are bonded, are a cycloalkane, a) for the preparation of an unsaturated ketone (I), is converted in the presence of a zeolite, of a phosphate having a zeolite structure and/or of a B, Ce, Fe, Zr or Sr phosphate as catalysts and b) for the preparation of a saturated ketone (II), is converted in the presence of hydrogen over a zeolite, a phosphate having a zeolite structure and/or a B, Ce, Fe, Zr or Sr phosphate, which carries one or more hydrogenation components, as catalysts.

**2.** A process as claimed in claim 1, wherein the catalyst used is a zeolite of the pentasil type, which may furthermore carry a hydrogenation component.

**3.** A process as claimed in claim 1, wherein the catalyst used is an aluminum silicate zeolite of the faujasite type, which may furthermore carry a hydrogenation component.

**4.** A process as claimed in any of claims 1 to 3, wherein the catalyst used is doped with an alkali metal, alkaline earth metal, a transition metal or a rare earth metal, if necessary in addition to the hydrogenation component.

**5.** A process as claimed in claim 1, wherein a hydrothermally prepared aluminum phosphate or silicon aluminum phosphate or silicon iron aluminum phosphate or iron aluminum phosphate or boron aluminum phosphate, which may furthermore carry a hydrogenation component, is used as the phosphate having a zeolite structure.

**6.** A process as claimed in any of claims 1 to 6, wherein the reaction is carried out in the gas phase.

## Revendications

**1.** Procédé de preparation de cétones insaturées de formule (I)

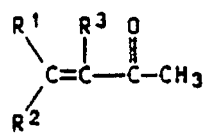

(I)

et de cétones saturées de formule (II)

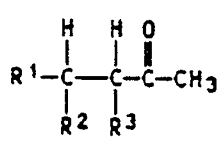

(II),

dans lesquelles $R^1$, $R^2$ et $R^3$ représentent des atomes d'hydrogène, des restes alkyle à 1-12 atomes de carbone, des restes cycloalkyle, aryle, aralkyle ou alkylaryle, qui peuvent être substitués à leur tour, ou bien $R^1$ et $R^3$ ou $R^2$ et $R^3$ peuvent former, avec l'atome de carbone auquel ils sont fixés, un groupement cycloalcane ou cycloalcène, caractérisé en ce qu'on fait reagir des 5-méthylène-1,3-dioxolannones substituées en position 4, de formule (III)

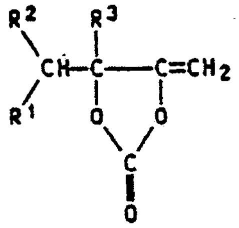

(III),

dans laquelle $R^1$, $R^2$ et $R^3$ représentent des atomes d'hydrogène, des restes alkyle à 1-12 atomes de carbone, des restes cycloalkyle, aryle, aralkyle ou alkylaryle, qui peuvent être substitués à leur tour, ou bien $R^1$ et $R^3$ ou $R^2$ et $R^3$ peuvent former, avec l'atome de carbone auquel ils sont fixés, un groupement cycloalcane,

a) en présence, en tant que catalyseur, de zéolithes, de phosphates de structure zéolithique et/ou de phosphates de B, de Ce, de Fe, de Zr ou de Sr, pour la préparation de cétones insaturées (I), et

b) en présence d'hydrogène et, en tant que catalyseur, de zéolithes, de phosphates de structure zéolithique et/ou de phosphates de B, de Ce, de Fe, de Zr ou de Sr qui portent au moins un composant d'hydrogénation, pour la préparation de cétones saturées (II).

**2.** Procédé selon la revendication 1, caractérisé en ce qu'on utilise, comme catalyseurs, des zéolithes du type pentasil qui portent en outre, s'il y a lieu, un composant d'hydrogénation.

**3.** Procédé selon la revendication 1, caractérisé en ce qu'on utilise, comme catalyseurs, des zéolithes d'aluminosilicate du type faujasite qui portent en outre, s'il y a lieu, un composant d'hydrogénation.

**4.** Procédé selon l'une quelconque des revendications 1 à 3, caractérisé en ce qu'on utilise des catalyseurs qui sont dopés avec des métaux alcalins, des métaux alcalino-terreux, des métaux de transition ou avec des terres rares, en plus du composant d'hydrogénation s'il y a lieu.

**5.** Procédé selon la revendication 1, caractérisé en ce qu'on utilise, comme phosphates de structure zéolithique, des phosphates d'aluminium ou des phosphates de silicium-aluminium ou des phosphates de silicium-fer-aluminium ou des phosphates de fer-aluminium ou des phosphates de bore-aluminium qui sont préparés par voie hydrothermale et qui portent en outre, s'il y a lieu, un composant d'hydrogénation.

**6.** Procédé selon l'une quelconque des revendications 1 a 6, caractérisé en ce qu'on conduit la reaction en phase gazeuse.